Europäisches Patentamt

European Patent Office

Office européen des brevets

(11) Numéro de publication: **0 240 643**
A1

## DEMANDE DE BREVET EUROPEEN

(21) Numéro de dépôt: 86400781.0

(22) Date de dépôt: **11.04.86**

(51) Int. Cl.⁴: **A61C 5/10** , A61C 13/083 , A61K 6/06

(43) Date de publication de la demande:
**14.10.87 Bulletin 87/42**

(84) Etats contractants désignés:
**CH DE FR LI**

(71) Demandeur: **Tyszblat, Michèle**
**3 Avenue Séverine**
**F-92400 Courbevoie(FR)**

(72) Inventeur: **Tyszblat, Michèle**
**3 Avenue Séverine**
**F-92400 Courbevoie(FR)**

(74) Mandataire: **Nony, Michel et al**
**Cabinet NONY & CIE 29, rue Cambacérès**
**F-75008 Paris(FR)**

(54) **Procédé de fabrication de prothèses dentaires.**

(57) Procédé de fabrication de prothèses dentaires pouvant constituer aussi bien des chapes périphériques que des "inlays" et "onlays" de reconstitution partielle consistant en : la prise sur un modèle d'une barbotine comportant de l'alumine, de l'eau et des additifs dont un liant ; la pre-consolidation de l'ébauche ainsi obtenue par un premier traitement thermique à environ 1.100°C de façon à obtenir un frittage par condensation-évaporation ; et la consolidation définitive par un deuxième traitement thermique à environ 1.100°C en présence de verre de façon à obtenir une infiltration du verre en fusion dans les pores de la pièce.

EP 0 240 643 A1

## Procédé de fabrication de prothèses dentaires".

La présente invention concerne un procédé de fabrication de prothèses dentaires ou de pièces céramiques, sans retrait après consolidation thermique (frittage), ces prothèses pouvant être ce qu'on appelle des chapes périphériques ou bien des "inlays" et "onlays" (pièces de reconstitution partielle).

Par chape périphérique, on désigne une prothèse qui recouvre complètement, en l'entourant, le reste d'une dent mis préalablement en forme par le praticien pour servir de support à cette prothèse. Dans ce cas, la réalisation de prothèse dentaire commence au cabinet dentaire par la préparation des dents ou ce qu'il en reste, pour obtenir une dépouille, au moyen d'instruments abrasifs, rotatifs ou autres. Cet usinage vise à rendre la préparation dentaire compatible avec l'insertion ultérieure de la prothèse qui restaurera l'organe dans son aspect dentaire et sa fonction. Cette prothèse peut être soit totalement métallique ou dans certains cas où l'esthétique est recherchée, elle peut être émaillée. Dans ce cas, on réalise une pièce support en général métallique qui coiffe la partie usinée de la dent, cette pièce support étant ensuite recouverte d'un émail. Cette pièce prothétique que l'on peut appeler chape ou infrastructure,est mise en forme par un procédé de coulée à cire perdue.

Les prothèses ainsi faites, appelées céramométalliques donnent satisfaction, mais la fabrication du support métallique est délicate et très onéreuse.

Par pièce de reconstitution partielle, on désigne une pièce qui est destinée à combler une cavité avec ou non recouvrement partiel de la dent. Dans ce cas la réalisation de la prothèse commence également au cabinet dentaire par la réalisation dans la dent d'une cavité présentant une dépouille. On réalise ensuite un moulage de la dent ainsi préparée, à partir duquel on prépare une pièce qui est destinée à être mise en place dans la cavité pour remplacer la portion de dent qui a été enlevée.

Ces pièces, appelées aussi "restaurations dentaires" présentent l'inconvénient de ne pas être très cathétiques ni parfaitement biocompatibles- (CORROSION).

La présente invention a pour objet un procédé permettant de réaliser des prothèses aussi bien de type chapes périphériques que de type pièces de reconstitution partielle en employant un mélange d'oxydes dont le composant majeur est l'alumine (85% en poids). Ce procédé permet de réaliser des prothèses dentaires totalement céramiques avec de très bonnes caractéristiques mécaniques et esthétiques ;la compatibilité biologique des céramiques et un coût de fabrication inférieur aux procédés actuels sont les autres avantages de ce procédé. Il est à noter que dans la pratique dentaire le terme "céramique" ne désigne pas uniquement les produits en terre cuite , mais les émaux et les oxydes métalliques tels que l'alumine.

Le procédé selon la présente invention est caractérisé par le fait qu'il comporte,au moins, la succession des trois opérations suivantes : dans un premier temps prise d'une barbotine à base d'alumine sur un modèle en matériau poreux ou non ; dans un deuxième temps,préconsolidation de l'ébauche ainsi obtenue par un traitement thermique à environ 1.100°, qui réalise un frittage sans retrait par condensation-évaporation; dans un troisième temps, consolidation définitive sans retrait de la pièce par infiltration de verre en fusion dans les pores de la pièce.

Suivant la nature de la prothèse que l'on veut obtenir, on ajoute à ces trois opérations essentielles diverses opérations de rectification ou finition.

Ainsi, entre le premier et le deuxième temps, on peut travailler l'ébauche en ajoutant ou en enlevant de la barbotine pour en modifier la forme.On peut également faire prendre la barbotine sur un modèle calcinable de façon à faire disparaître ce modèle lors du premier traitement thermique de pré-consolidation.On peut faire prendre la barbotine sur un modèle non calcinable et obtenir la séparation par simple séchage de la barbotine.On peut également obtenir la prise de la barbotine en employant un modèle poreux de façon à provoquer une succion de l'eau contenue dans la barbotine.

L'invention sera mieux comprise à l'aide des exemples ci-après qui sont donnés à titre purement indicatif et non limitatif.

Exemple I. Réalisation d'une chape périphérique.

On commence, comme cela est connu, par l'usinage de la ou des dents puis, on réalise des empreintes qui constituent une réplique en négatif des dents préparées, adjacentes et antagonistes. Puis, comme cela est connu également, le technicien de laboratoire ou le dentiste réalise au moyen d'une coulée à l'intérieur de l'empreinte une réplique en positif que l'on appelle le modèle de travail. Dans le cas présent le procédé selon l'invention nécessite cependant la réalisation d'une deuxième réplique positive de la ou des dents préparées. L'une au moins des deux répliques en positif devra obligatoirement être réalisée en un matériau poreux (du plâtre par exemple) pour la mise en oeuvre du procédé.

On dispose ainsi :

-du modèle de travail comprenant les dents préparées, antagonistes , adjacentes ;

-d'un modèle positif en plâtre ou autre matériau poreux de la ou des dents préparées.

La réplique en matériau poreux est alors immergée dans une barbotine constituée d'un mélange d'eau de poudre d'alumine et d'additifs dont un liant.Une succion de l'eau par les capillaires du plâtre provoque une agglomération des grains d'alumine à la surface du modèle. On obtient ainsi une couche d'alumine, homothétique à la forme du modèle en plâtre. L'épaisseur de cette couche est déterminée en fonction du temps d'immersion de la réplique en matériau poreux dans la barbotine et cette épaisseur est de façon générale, comprise entre 0,5 et 1 millimètre, ou plus si on le désire.

Le technicien de laboratoire peut alors modifier ce dépôt par apport local de barbotine à l'aide d'un pinceau ou d'une spatule. Le dépôt a la consistance d'une glaise,de sorte qu'il est possible de le sculpter pour effectuer les finitions nécessaires . Cette opération étant terminée, l'ensemble est mis à sécher à l'air ou, pour accélérer le séchage dans une étuve sous vide primaire ou sous faible pression partielle de vapeur d'eau. On réalise ensuite un premier traitement thermique à 1.100°C environ (plus ou moins 5ø°C) que entrainera d'une part la séparation de la chape en alumine de son support en matériau poreux et d'autre part la pré-consolidation de cette chape. En effet la réplique en plâtre , lors de la montée en température, se déshydrate et se transforme en sulfate de calcium anhydre. Cette déshydratation et cette transformation s'accompagnent d'une importante rétraction volumique. A la fin de ce traitement thermique, le modèle en plâtre est donc détruit. Ce traitement a aussi pour but de consolider la chape en alumine pour la rendre manipulable. Cette pré-consolidation s'effectue sans retrait, en favorisant les mécanismes de frittage sans retrait , qui sont connus des métallurgistes sous le nom de condensation-évaporation. On dispose alors d'une chape en alumine, poreuse , pré-consolidée, mais dont les caractéristiques mécaniques sont insuffisantes. On procède alors à un deuxième traitement thermique pour obtenir la consolidation totale sans retrait. Dans ce but, on réalise l'infiltration d'un verre à l'état liquide, dans les capillaires de la chape en alumine poreuse . Une quantité adaptée au volume poreux de la chape en alumine est mise en contact avec elle, l'ensemble est monté en température (1.100°C + ou - 5ø°C) , le verre fond et s'infiltre par capillarité dans les pores de la chape. Le verre utilisé est choisi pour ses caractéristiques de : tension superficielle , viscosité à la température d'infiltration et coefficient de dilatation.

Ce traitement thermique terminé, la chape est prête à être utilisée et dans les cas où on recherche une esthétique plus poussée, à être émaillée.

Pour cette opération, on utilise la deuxième réplique que l'on coiffe avec la chape ainsi fabriquée et que l'on revêt, selon les techniques connues du produit destiné à réaliser l'émaillage. Cette opération étant connue n'est pas décrite de façon détaillée.

Exemple II - Réalisation d'une chape périphérique sans émaillage.

On opère comme précédemment, mais on ne réalise qu'une seule réplique, en matériau poreux. Comme dans l'exemple précédent,on immerge cette réplique dans la barbotine, mais en la laissant immergée pendant un temps nettement plus long de façon à obtenir le dépôt d'une couche de barbotine d'une épaisseur importante,de l'ordre de 3 millimètres environ. Le technicien de laboratoire peut alors modifier la forme de ce dépôt, mais dans ce cas, il opère essentiellement en retirant le matériau en excès de façon à sculpter la pièce obtenue pour lui donner la forme extérieure d'une dent.

Il suffira alors de procéder aux deux opérations de pré-consolidation et ensuite de consolidation définitive au verre pour obtenir directement, sans émaillage ultérieur, une prothèse prête à être mise en place.

Dans ce cas, il est préférable de mettre un additif colorant dans le verre pour obtenir un aspect extérieur satisfaisant au point de vue esthétique.

Exemple III. Réalisation d'une pièce de reconstitution partielle. On commence, comme cela est connu, par l'usinage de la dent de façon à ménager une cavité présentant une dépouille ;puis on réalise une empreinte qui constitue une réplique en négatif de la dent ainsi préparée. Ensuite toujours de façon connue, on réalise au moyen d'une coulée à l'intérieur de l'empreinte, une réplique en positif.

Dans cette réplique en positif ,on dépose, au moyen d'une spatule une quantité suffisante de barbotine que l'on sculpte et met en forme de façon à reconstituer la partie manquante de la dent.

Si la réplique est en matériau poreux calcinable ,ou non poreux calcinable, on procède comme dans les deux exemples précédents,c'est-à-dire que le premier traitement thermique de pré-consolidation provoque la destruction du modèle.

Si la réplique est en matériau non calcinable, on procède à un séchage soit à l'air libre, soit par ventilation d'air chaud pour faire évaporer l'eau de la barbotine de façon à obtenir la pièce dont les particules tiennent ensemble. La pièce est séparée de la réplique ; puis on fait subir à la pièce ainsi obtenue le premier traitement thermique de pré-consolidation.

Dans un cas comme dans l'autre, on dispose , à la fin du premier traitement thermique, d'une pièce pré-consolidée à laquelle on fait subir le deuxième traitement thermique en la mettant en contact avec du verre en fusion qui s'infiltre dans les pores de ladite pièce.

Après ce deuxième traitement thermique, on dispose de la pièce définitive que l'on met en place dans la cavité dentaire.

On obtient ainsi une reconstitution partielle de la dent qui est plus solide mécaniquement et beaucoup plus résistante biochimiquement que tous les "inlays" et "onlays" actuellement employés.

De préférence, la barbotine dans laquelle on plonge la réplique en matériau poreux est constituée de la façon suivante.

87,6 % ± 5 % en poids d'alumine,
12,3 % ± 5 % en poids d'eau,
0,1 % en poids d'un liant.

D'autre part, pour la vitrification, on utilise par exemple un verre constitué par :

32 % en poids de $SiO_2$
30 % en poids de $B_2O_3$
12 % en poids de $Al_2O_3$
10 % en poids de $Na_2O$
12 % en poids de CaO
4% en poids de $Ti_o2$

Dans cette composition l'oxyde de titane sert de colorant ; on peut donc avantageusement remplacer un peu de $Ti_o2$ par d'autres oxydes colorants.

D'autre part, cette composition de verre n'est donnée qu'à titre d'exemple non limitatif , car on peut employer une grande variété de compositions de verre.

En particulier dans cet exemple, on peut faire varier la proportion d'alumine entre 10 % et 20 % en jouant de façon correspondante sur les proportions des autres constituants.

## Revendications

1. Procédé de fabrication de prothèses dentaires pouvant constituer aussi bien des chapes périphériques que des "inlays" et "onlays" de reconstitution partielle, caractérisé par le fait que lesdites prothèses sont obtenues en procédant au moins aux trois opérations successives suivantes :

a-prise sur un modèle d'une barbotine comportant de l'alumine, de l'eau et des additifs dont un liant ;

b-pré-consolidation de l'ébauche ainsi obtenue par un premier traitement à environ 1.100°C (+ ou -50°C) de façon à obtenir un frittage sans retrait par condensation-évaporation :

c-consolidation définitive sans retrait par un deuxième traitement thermique à environ 1.100°C (+ ou -50°C) en présence de verre de façon à obtenir une infiltration du verre en fusion dans les pores de la pièce.

2. Procédé selon la revendication 1, selon lequel on procède à la modification de l'ébauche après prise de la barbotine sur le modèle soit en effectuant un apport de barbotine complémentaire, soit en enlevant de façon à lui donner la forme désirée.

3. Procédé selon la revendication 1 ou 2,selon lequel on réalise la séparation de l'ébauche et de son support par calcination du support.

4. Procédé selon la revendication 3, dans lequel le support calcinable est poreux de façon que la prise de la barbotine se fasse par succion de l'eau. 0\

5. Procédé selon la revendication 1 ou 2, selon lequel on réalise la séparation de l'ébauche et de son support par séchage de l'ébauche.

6. Procédé selon l'une quelconque des revendications 1 à 4, selon lequel on réalise,de façon connue,une préparation mécanique de la dent pour obtenir une dépouille suivie de la prise d'une empreinte en négatif, caractérisé par le fait qu'on réalise ensuite deux répliques en positif dont une au moins doit être en matériau poreux ;puis on plonge la réplique en matériau poreux dans une barbotine constituée d'un mélange d'eau, d'alumine et d'un liant de façon à se recouvrir d'une couche d'alumine s('agglomérant à la surface de la réplique par effet de succion de l'eau ; puis on fait subir à la réplique en matériau poreux ainsi recouverte un traitement thermique à environ 1.100°C au cours duquel la réplique en matériau poreux est détruite et l'alumine agglomérée est pré-consolidée par frittage de façon à former une chape creuse poreuse; puis on consolide sans retrait cette chape par imprégnation par capillarité de ses pores au moyen de verre fondu à environ 1.100°C;ce après quoi ladite chape peut être recouverte d'émail par tout moyen connu.

7. Procédé selon l'une quelconque des revendications 1 à 4,selon lequel on réalise, de façon connue, une préparation mécanique de la dent pour obtenir une dépouille , suivie de la prise d'une empreinte en négatif elle-même suivie de la réalisation d'une réplique en positif en matériau poreux , caractérisé par le fait qu'on plonge la réplique en matériau poreux dans une barbotine constituée d'un mélange d'alumine, d'eau et de liant pendant un temps suffisant pour obtenir le dépôt sur la réplique en positif d'un dépôt épais de barbotine ; puis on sculpte l'ébauche ainsi obtenue de façon à lui donner la forme extérieure définitive de la prothèse à obtenir ;ce après quoi on lui fait subir les deux traitements thermiques de pré-consolidation et de consolidation définitive en obtenant ainsi directement , sans émaillage, la chape périphérique définitive.

8. Procédé selon la revendication 7, selon lequel on ajoute un colorant au verre de consolidation définitive.

9. Procédé selon l'une quelconque des revendications 1 à 5, selon lequel on ménage dans la dent de façon connue, une cavité présentant une dépouille dont on prend une empreinte en négatif pour réaliser ensuite un modèle positif,caractérisé par le fait qu'on réalise dans la cavité de ce modèle la prise d'une barbotine constituée par un mélange d'alumine, d'eau et de liant de façon à obtenir une ébauche ayant la forme de la pièce finale de reconstitution partielle que l'on veut obtenir ;ce après quoi on fait subir à ladite ébauche les deux traitements thermiques de pré-consolidation et de consolidation définitive en obtenant l' "inlay" ou l' "onlay" de reconstitution partielle que l'on peut mettre en place dans la cavité de la dent.

10. Procédé selon l'une quelconque des revendications précédentes dans lequel, dans la barbotine, la proportion d'alumine est au moins de 85 % en poids.

11. Procédé selon la revendication 10, dans lequel la barbotine est composée de :
87,6 % ± 5 % en poids d'alumine,
12,3 % ± 5 % en poids d'eau,
0,1 % en poids de liant

12. Procédé selon l'une quelconque des revendications 1 à 11, dans lequel le verre est composé de :
32 % en poids de $SiO_2$
30 % en poids de $B_2O_3$
12 % en poids de $Al_2O_3$
10 % en poids de $Na_2O$
12 % en poids de CaO
4% en poids de $Ti_o2$

13. Procédé selon la revendication 12, dans lequel la proportion d'oxyde de Titane peut être diminuée en le remplaçant partiellement par des oxydes colorants.

14. Procédé selon la revendication 12 ou 13 dans lequel la proportion d'alumine peut varier de 10 % à 20 % en poids,les proportions des autres constituants étant ajustées en conséquence.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-B-1 491 042 (NATIONAL RESEARCH DEVELOPMENT CORP.) * colonne 4, ligne 3 - colonne 5, ligne 50; colonne 8, ligne 38 - colonne 10, ligne 37 * | 1 | A 61 C 5/10 A 61 C 13/083 A 61 K 6/06 |
| | --- | | |
| A | FR-A-2 129 058 (CERAVER) * revendications 1, 2 * | 1 | |
| | --- | | |
| A | DE-C- 216 213 (FRITZSCHE) * page 2, lignes 5-26 * | 1 | |
| | --- | | |
| A | US-A-4 040 844 (GNECCO) * colonne 4, ligne 43 - colonne 5, ligne 9 * | 1 | |
| | --- | | **DOMAINES TECHNIQUES RECHERCHES (Int. Cl.4)** |
| A | CH-A- 428 088 (NATIONAL RESEARCH DEVELOPMENT CORP.) * colonne 7, ligne 45 - colonne 8, ligne 33 * | 1 | A 61 C 5/00 A 61 C 13/00 A 61 K 6/00 |
| | --- | | |
| A | FR-A-2 291 736 (LCC-CICE) * page 3, lignes 5-10 * | 1 | |
| | --- | | |
| A | CERAMIC ENGINEERING AND SCIENCE PROCEEDINGS, vol. 6, janvier/février 1985, page 1-9, Columbus, Ohio, US; J.W. MCLEAN: "Current status and future of ceramics in dentistry" | | |
| | ----- | | |

Le présent rapport de recherche a été établi pour toutes les revendications

| Lieu de la recherche | Date d'achèvement de la recherche | Examinateur |
|---|---|---|
| BERLIN | 28-11-1986 | SIMON J J P |